(19) Europäisches Patentamt / European Patent Office / Office européen des brevets

(11) **EP 3 892 313 B1**

(12) **EUROPEAN PATENT SPECIFICATION**

(45) Date of publication and mention of the grant of the patent:
**11.01.2023  Bulletin 2023/02**

(21) Application number: **20168991.6**

(22) Date of filing: **09.04.2020**

(51) International Patent Classification (IPC):
**A61M 1/34** $^{(2006.01)}$

(52) Cooperative Patent Classification (CPC):
**A61M 1/3437; A61M 1/3441; A61M 1/3455;**
A61M 2202/0007; A61M 2202/0486

(54) **APPARATUS FOR EXTRACORPOREAL BLOOD TREATMENT**

VORRICHTUNG ZUR EXTRAKORPORALEN BLUTBEHANDLUNG

APPAREIL DE TRAITEMENT SANGUIN EXTRACORPOREL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**13.10.2021  Bulletin 2021/41**

(73) Proprietor: **Gambro Lundia AB**
**226 43 Lund (SE)**

(72) Inventor: **ROVATTI, Paolo**
**41034 Finale Emilia (IT)**

(74) Representative: **PGA S.p.A., Milano, Succursale di Lugano**
**Via Castagnola, 21c**
**6900 Lugano (CH)**

(56) References cited:
**EP-A1- 2 644 215    US-A1- 2012 143 116**

EP 3 892 313 B1

**Description**

TECHNICAL FIELD

The present invention relates to an apparatus for extracorporeal blood treatment for administering nutritional products.

Extracorporeal blood treatment involves removing blood from a patient, treating the blood externally to the patient, and returning the treated blood to the patient. Extracorporeal blood treatment is typically used to extract undesirable matter or molecules from the patient's blood and add desirable matter or molecules to the blood. Extracorporeal blood treatment is used with patients unable to effectively remove matter from their blood, such as when a patient has suffered temporary or permanent kidney failure. These patients and other patients may undergo extracorporeal blood treatment to add or remove matter to their blood, to maintain an acid/base balance or to remove excess body fluids, or to perform extracorporeal gas exchange processes, for example.

In particular, the invention relates to chronic (long-term) hemodialysis (HD) therapy systems used to treat patients with chronic kidney failure. HD therapy is usually performed several times a week (e.g. 3 times a week) and each treatment has an average duration of some hours (e.g. 3.5 - 4.5 hours). During the therapy, the patient has two accesses (arterial/vein) where blood flows in/out from the body, wherein the average blood flow is usually between 250 and 400 ml/min.

BACKGROUND OF THE INVENTION

The apparatuses for chronic (long-term) hemodialysis (HD) therapy comprise a device for online preparation of the dialysis liquid and/or replacement liquid. The device comprises one, two or more containers of concentrate located on respective injection lines which are predisposed to supply substances, such as electrolytes, buffer agents or others, towards a preparation line connected to a water source and located upstream a dialysis line. The concentrate containers may comprise concentrates in the liquid state or solid state, for example powder.

In patients receiving chronic hemodialysis, the National Kidney Foundation currently recommends a daily protein intake of 1.2 g/kg or more in patients undergoing hemodialysis. When malnutrition is present, a stepwise approach to treatment is used, beginning with dietary counseling and diet modifications, followed by oral nutritional supplements, and then by enteral nutrition supplements (usually consisting of a mixture of amino acids, glucose, and lipids) or parenteral nutritional supplements if needed.

In hemodialysis, it is known to administer IntraDialytic Parenteral Nutrition (IDPN) infusion e.g., through a venous access into the patient, typically, some minutes after dialysis has begun, and continued throughout the remainder of a dialysis session.

This is done by means of dedicated devices, such a bag, a dedicated line and a pump which are operated manually. This requires additional nurse workload.

In addition, the dialysis therapy parameters are corrected manually on the base of the operator experience, measurements or knowledge of the additional fluid/weight it is infused in this way. The manual correction of the dialysis therapy parameters does not allow to calculate in accurate manner the mass balance of the patient.

This is also a cumbersome and invasive solution and it is not usually well accepted by the patients/users.

An aim of the present invention is to provide for an apparatus for extracorporeal blood treatment that alleviates or minimizes or remedy the above-mentioned drawbacks.

It is an aim of the present description to provide an extracorporeal blood treatment apparatus and a method for administering nutritional products in an apparatus for extracorporeal blood treatment which are able to take into account the administered nutritional products to properly manage the mass balance of the patient during treatment.

It is an aim of the present invention to better control the administration of the nutritional products during therapy.

It is a further aim of the present description to control administration of the nutritional products through the same apparatus for extracorporeal blood treatment.

It is a further aim of the present description to reduce the wrkload of nurses.

It is a further aim of the present description to reduce complexity of devices dedicated to extracorporeal blood treatment with simultaneous administration of nutritional products.

It is a further aim to improve the comfort of patients undergoing treatment.

Document US5776345 is also known. This document discloses machine for acute treatment provided with a blood circuit to which a plurality of infusion lines may infuse fluids. A dialysis fluid container, a collection fluid container, a replacement fluid container and an anticoagulant container are connected to scales which weigh the contents. The replacement fluid adds material to the blood in order to adjust the pH of the blood, to add nutrients to the blood or to add fluid to the blood.

Document US2012143116 discloses a renal failure therapy system including a blood pump, a dialysis fluid pump, a filtrate pump, at least one infusion pump, and a control unit configured to synchronize operation of the infusion pump to the blood pump and the filtrate pump. In particular, the renal failure therapy system allows external infusion, IV or administration pumps to be synchronized with the internal pumps of the medical fluid therapy machine and automatically adjusts for variations in flowrate of one fluid with respect to another. For example, filtrate/output pump internal to the machine may increase or decrease in response to the flowrate or output rate of a manually entered value of an external pump or measuring device. In short, the system provides a more "hands-off", safe and effective method and apparatus for medical fluid therapy delivery and removal.

Document EP2644215 discloses an intensive care apparatus for extracorporeal treatment of blood with a filtration unit, a blood circuit, a pre and/or post-dilution fluid line connected to the blood circuit, and a dialysis circuit. Pumps act on the fluid lines for regulating the flow of fluid. A control unit is configured to periodically calculate a new value for the patient fluid removal rate to be imposed on an ultrafiltration actuator in order to keep a predefined patient fluid removal rate across a reference time interval irrespective of machine down times. The apparatus presents an infusion line connected with the blood withdrawal line upstream the blood pump and with an infusion fluid container, which contain a drug, or a regional anticoagulant, or a nutrients solution or other. This infusion line is referred to as pre-blood pump infusion line.

The invention is defined by the features of independent claims 1 and 2.

An apparatus according to one or more of the appended claims, taken singly or in any combination, attains at least one of the above-indicated aims.

Further characteristics of the present invention will better emerge from the detailed description that follows of some embodiments of the invention, illustrated by way of non-limiting examples in the accompanying figures.

DESCRIPTION OF THE DRAWINGS

The description will now follow, with reference to the appended Figures, provided by way of non-limiting example, in which:

Figure 1 shows a front view of an extracorporeal blood treatment apparatus according to the invention;
Figure 1a shows a disposable nutritional line;
Figures 2 schematically shows the extracorporeal blood treatment apparatus of figure 1;
Figure 3 shows an enlarged portion of the extracorporeal blood treatment apparatus of figure 2;
Figure 4 shows a portion according to a variant embodiment of the apparatus of figures 2 and 3;
Figures 5 is a flow diagram of a method as such not falling under the claimed invention for administering nutritional products according to the invention.

DETAILED DESCRIPTION

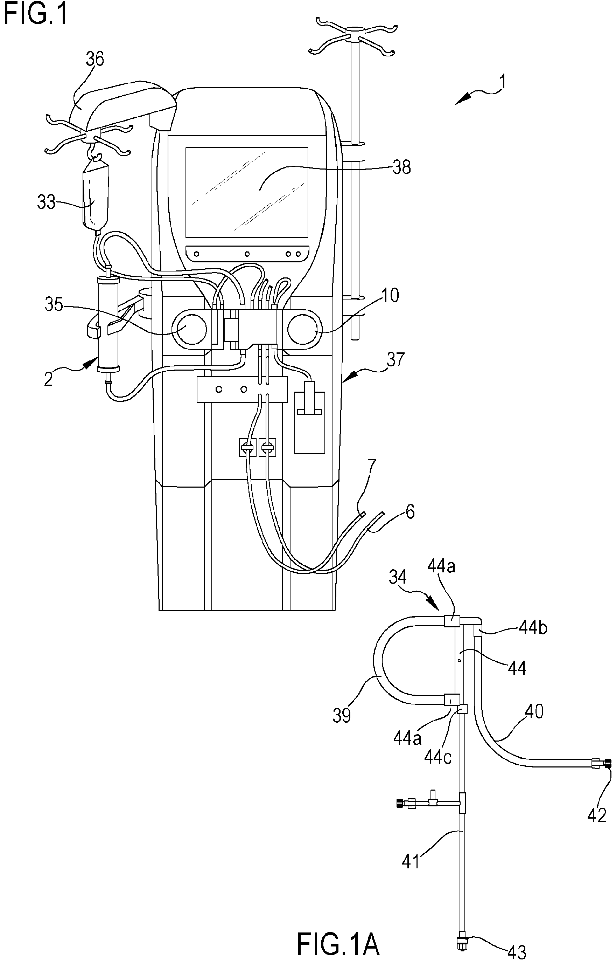

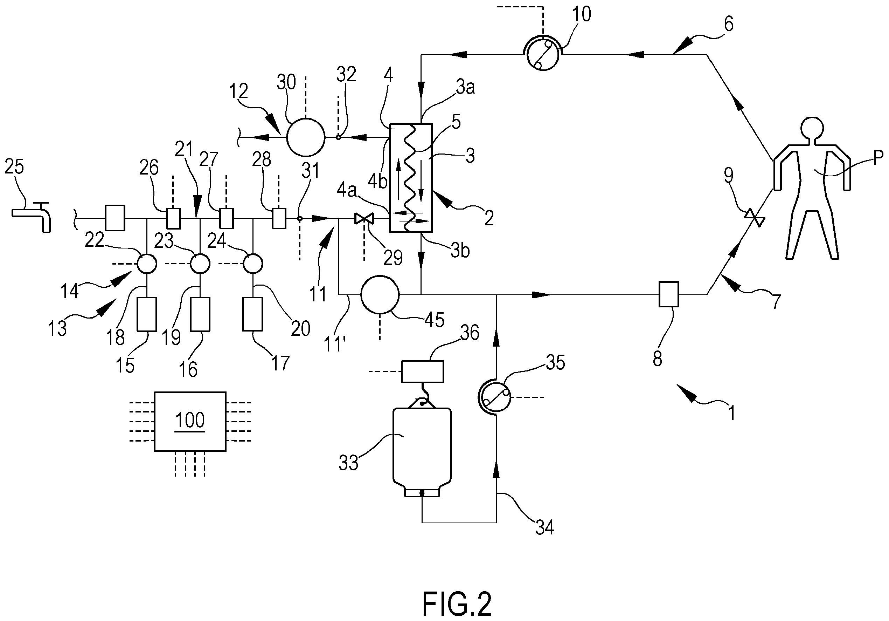

An apparatus 1 for extracorporeal blood treatment for chronic (long term) therapy is represented in Figures 1 and 2. The apparatus 1 comprises a filtration unit 2 having a primary chamber 3 and a secondary chamber 4 separated by a semi-permeable membrane 5. Depending upon the treatment, the semi-permeable membrane 5 of the filtration unit 2 may be selected to have different properties and performances.

A blood circuit is coupled to the primary chamber 3 of the filtration unit 2. The blood circuit comprises a blood withdrawal line 6 connected to an inlet 3a of the primary chamber 3, a blood return line 7 connected to an outlet 3b of the primary chamber 3. The withdrawal line 6 and blood return line 7 are configured for connection to a cardiovascular system of a patient "P".

In use, the blood withdrawal line 6 and the blood return line 7 are connected to a needle or to a catheter or other access device which is then placed in fluid communication with the patient "P" vascular system, such that blood may be withdrawn through the blood withdrawal line 6, flown through the primary chamber 3 and then returned to the patient's vascular system through the blood return line 7. An air separator, such as a deaeration chamber 8, may be present on the blood return line 7. Moreover, a monitor valve 9 may be present on the blood return line 7, downstream the deaeration chamber 8.

The blood flow through the blood circuit is controlled by a blood pump 10, for instance a peristaltic blood pump, acting either on the blood withdrawal line 6 or on the blood return line 7. The embodiment of Figure 2 shows the blood pump 10 coupled to a pump section of the withdrawal line 6. A control unit 100 is connected and controls the blood pump 10 to regulate a blood flow rate.

A dialysis circuit is connected to the secondary chamber 4 of the filtration unit 2 and comprises a dialysis supply line 11 connected to an inlet 4a of the secondary chamber 4 and a dialysis effluent line 12 connected to an outlet 4b of the secondary chamber 4 and to a drain, not shown.

The dialysis supply line 11 is connected to a preparation device 13 for preparing a fresh dialysis fluid. The preparation device 13 comprises a regulating device 14 for regulating the composition of the fresh dialysis fluid. The dialysis effluent line discharges a spent dialysis fluid into the drain.

In the example of Figure 2, the preparation device 13 comprises three containers of concentrate 15, 16, 17 located on respective injection lines 18, 19, 20 which are predisposed to supply substances such as electrolytes, buffer agents or others towards a preparation line 21 of the fresh dialysis fluid. The concentrate containers 15, 16, 17 may comprise concentrates in the liquid state or solid state, for example powder.

The regulating device 14 comprises injection pumps 22, 23, 24 placed on the injection lines 18, 19, 20 to move the fluid along the respective injection line 18, 19, 20 towards the preparation line 21 which collects the liquid, for example water, from a source 25. The preparation line 21 is located upstream the dialysis supply line 11 and has one end connected to the source 25, e.g. a deionized/purified water source or a reverse osmosis water plant, and an opposite end connected to the dialysis supply line 11. The source 25 may comprise the water source, as shown, or a source of ultra-pure liquid.

Concentration or conductivity sensors 26, 27, 28 are located on the preparation line 21 and are able to provide the control unit 100 with a signal related to conductivity or concentration of a predetermined substance (for example sodium) of the fluid crossing the preparation line 21 such that the control unit 100 is able to control the injection pumps 22, 23, 24 in order to regulate the conductivity Cd or concentration, for example of sodium [Na], of the liquid crossing the dialysis supply line 11. A fluid check organ 29 may be used to selectively enable or inhibit a passage of fluid across the dialysis line 21 and into the filtration unit 2 in case the liquid does not meet the required parameters.

An infusion line 11' departs from the dialysis supply line 11 and is connected to the blood return line 7 to infuse part of the fresh dialysis fluid into the blood circuit.

An auxiliary infusion pump 45 may be coupled to the infusion line 11' to deliver said part of the fresh dialysis fluid into the blood circuit.

Of course the infusion line 11' may alternatively or in addition being connected to the blood withdrawal line 6 (in particular downstream the blood pump 10) for pre-infusing a substitution fluid.

An ultrafiltration device is configured to achieve a fluid removal from the patient through the semi-permeable membrane 5 of the filtration unit 2. The ultrafiltration device comprises a dialysis pump 30 located on the dialysis effluent line 12. In a variant embodiment, a first dialysis pump is coupled to the dialysis supply line 11 and a second dialysis pump coupled to the dialysis effluent line 12. A first flow-meter 31 is active on the dialysis supply line 11 and is placed between the fluid check organ 29 and the inlet 4a of the secondary chamber 4. A second flow-meter 32 is active on the dialysis effluent line 12 and is placed between the outlet 4a of the secondary chamber 4 and the dialysis pump 30.

The infusion line 11' is connected to the dialysis supply line 11 between the first flow-meter 31 and the inlet 4a of the secondary chamber 4. The first flow-meter 31 and the second flow-meter 32 are connected to the control unit 100 and are configured to determine an ultrafiltration rate UFR.

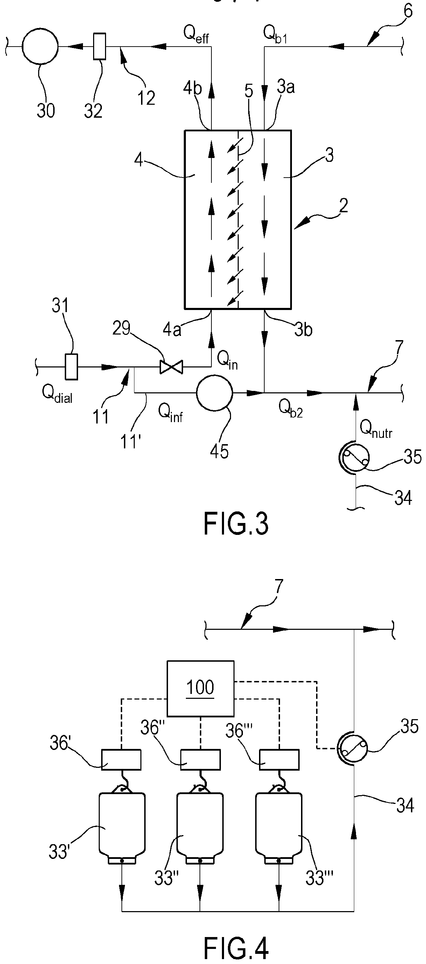

The ultrafiltration rate UFR is a difference between the spent dialysis fluid exiting the outlet 4b of the secondary chamber 4 and the prepared fresh dialysis fluid routed to the inlet 4a of the secondary chamber 4 and infused into the blood circuit through the infusion line 11' (figure 3).

As shown in figure 3, $Q_{b1}$ is the blood flow rate entering the primary chamber 3 through the inlet 3a, $Q_{b2}$ is the blood flow rate leaving the primary chamber 3 through the outlet 3b, $Q_{in}$ is the flow rate entering the secondary chamber 4 through the inlet 4a, $Q_{inf}$ is the flow rate crossing the infusion line 11', wherein:

$$Q_{dial} = Q_{in} + Q_{inf}$$

The first flow-meter 31 and the second flow-meter 32 provide the control unit 100 with an instant value of the respective flows and thus enable the control unit 100 to calculate an instant ultrafiltration rate UFR. Alternatively, a differential sensor may be provided, active on the dialysis supply line 11 and on the dialysis effluent line 12 and therefore able directly to provide a signal relating to the ultrafiltration rate UFR. Instead of flowmeters, balance chambers may operatively be coupled to the dialysis circuit is provided. The balance chambers principle operates so that the amount of fluid entering into the first chamber on the dialysis supply line 11 is equal to the amount of fluid exiting from the dialysis effluent line 12.

To achieve the ultrafiltration, an ultrafiltration line (not represented) is added to the effluent line upstream the balancing chamber. An ultrafiltration pump removes the desired amount of ultrafiltered liquid before that the spent dialysis liquid reaches the second balance chamber thereby achieving an ultrafiltered volume. In this alternative embodiment, the second parameter is related to the amount of liquid volume that is removed through the ultrafiltration line and in particular is the ultrafiltration rate through the ultrafiltration line. Of course absolute volume variation through ultrafiltration line may be measured too.

The apparatus 1 further comprises one nutritional bag 33 containing a nutritional solution and a nutritional line 34 having a first end in fluid communication with the nutritional bag 33 and a second end connected to the blood return line 7 for infusing the nutritional solution into the patient vascular system through said blood return line 7. In an alternative embodiment, the nutritional solution may be infused directly into the patient vascular system. An infusion pump 35 is coupled to the nutritional line 34 to deliver the nutritional solution through the nutritional line 34. A sensing element in the form of a weighing device 36 is configured to weigh the nutritional bag 33 while the nutritional solution is infused and to provide a first signal allowing calculation of a first parameter W related to a weight of the nutritional bag 33 and, therefore, to the weight or volume of nutritional solution contained in the nutritional bag 33. In general, the weighing device 36 provides the time variation of the bag weight which is directly linked to the actual flow rate of the nutritional solution through the nutritional line. Indeed, the control unit knows the nutritional line cross section and may therefore easily and very accurately calculate the actual flow rate of the nutritional solution through the nutritional line starting from measurements of weight/weight variation over time of the corresponding bag. Optionally the first parameter is the weight of the nutritional bag 33 measured over time.

As an alternative, a flow meter on the nutritional line may be used as a sensing element.

Finally, though less precise, the pump speed may be used to determine flow rate through the nutritional line, the speed being monitored through a suitable sensor, such as a Hall sensor or a sensor sensitive to pump electric parameters such as resistance or power consumption.

As shown in Figure 1, the apparatus 1 comprises a main body 37 provided with a base resting on the ground and hosting all the components of said apparatus 1, i.e.: the filtration unit 2, the blood circuit, the blood pump 10, the dialysis circuit, the preparation device 13, the weighing device 36, the nutritional line 34, the infusion pump 35, the control unit 100. The weighing device 36 is a weight scale and is configured to hang the nutritional bag 33.

The control unit 100 is housed in the main body 37. The infusion pump 35 and the blood pump 10 are peristaltic pumps supported by the main body 37. Each peristaltic pump comprises an actuator or motor, not shown, connected to a rotor. The rotor of the infusion pump 35 and the rotor of the blood pump 10 are placed on a front face of the main body 37.

The blood circuit and the filtration unit 2 are disposable (i.e. they are disposed after each blood treatment) and are coupled in removable manner to the main body 37. The pump section of the withdrawal line 6 is coupled in removable manner to the rotor of the blood pump 10. The nutritional line 34 is disposable and is coupled in removable manner to the main body 37. A pump section of the nutritional line 34 is coupled in removable manner to the infusion pump 35.

Figure 1A shows an example of disposable nutritional line 34. The nutritional line 34 comprises a pump segment 39, a feeding tube segment 40 connected to an inlet of the pump segment 39, and a delivery tube segment 41 connected to an outlet of the pump segment 39.

In particular, the pump segment 39 may have a passage section for the nutritional solution larger than a passage section of the delivery tube segment 41 and/or than a passage section of the feeding tube segment 40. In general, the passage section of the delivery tube segment is equal to the passage section of the feeding tube segment.

On a first end for connection to the nutritional bag (or to a tubing connected to the nutritional bag), the feeding tube segment 40 includes a respective removable connector 42, in particular a Luer connector. The other end is joined to a rigid connector 44b associated to a rigid portion 44 of the nutritional line 34 supporting the pump segment 39. Correspondingly, the delivery tube segment 41 includes a respective removable connector 43, in particular a Luer connector, connectable to a corresponding counter connector on the blood return line 7, for example at the deareation chamber 8.

As mentioned, the nutritional line further comprises one rigid portion 44 (the portion being more rigid than the flexible feeding and delivery tube segments 40, 41) comprising two pump connectors 44a for receiving opposite ends of the pump segment, the delivery tube segment connector 44c for receiving one end of the delivery tube segment 41 and the feeding tube segment connector 44b for receiving one end of the feeding tube segment 40.

The extracorporeal blood treatment apparatus main body 37 includes a coupling device; the rigid body 44 is configured to couple with the apparatus coupling device to position the nutritional line on the main body in specific arrangement with respect to the infusion pump so that the pump segment may be precisely received around the infusion pump rotor. In particular the coupling device is placed on the front panel of the main body 37.

The dialysis circuit is not-disposable and is configured to be sterilized after each blood treatment. Tubes, pumps, sensors of the dialysate circuit are not configured to be replaced after each treatment but only for maintenance purposes or in case of faults. Therefore, the dialysis circuit is integrated in the main body 36 and mounted fixed in or on the main body 37. The dialysis pump 30 is a volumetric pump crossed by the effluent fluid and is mounted in the main body 37.

The control unit 100 is contained in or supported by the main body 37. The control unit 100 is connected to the blood pump 10, the weighing device 36, the infusion pump 35, the injection pumps 22, 23, 24 of the regulating device 14, the concentration or conductivity sensors 26, 27, 28, the first flow-meter 31, the second flow-meter 32, the fluid check organ 29 and to the dialysis pump 30 of the ultrafiltration device. The control unit 100 controls the weighing device to measure the weight at instants of time, e.g. with an acquisition frequency 1 Hertz. The control unit 100 is also connected to a display screen 38 (Figure 1) mounted on the main body 37.

The control unit 100 may comprise a digital processor (CPU) with memory (or memories), an analogical type circuit, or a combination of one or more digital processing units with one or more analogical processing circuits. In the present description and in the claims it is indicated that the control unit 100 is "configured" or "programmed" to execute steps: this may be achieved in practice by any means which allow configuring or programming the control unit 100. For instance, in case of a control unit 100 comprising one or more CPUs, one or more programs are stored in an appropriate memory: the program or programs containing instructions which, when executed by the control unit 100, cause the control unit 100 to execute the steps described and/or claimed in connection with the control unit 100. Alternatively, if the control unit 100 is of an analogical type, then the circuitry of the control unit 100 is designed to include circuitry configured, in use, to process electric signals such as to execute the control unit 100 steps herein disclosed.

The control unit 100 is configured to or programmed for receiving signals from the sensors and from other inputs and for commanding pumps and valves according to said signals, in order to perform the blood treatment and to administer nutritional products to the patient during blood treatment. The control unit 100 is also configured to or programmed for displaying data on the display screen 38.

The nutritional solution contained in the nutritional bag 33 comprises a mixture of amino-acids, glucose or dextrose and lipids. For instance, the solution consists of 300 ml of amino-acids at a concentration of 15%, 150 ml of dextrose at a concentration of 50% and 150 ml of lipids at a concentration of 20%. The amount of nutritional solution contained in the nutritional bag 33 may be the total amount administered to the patient at the end of the blood treatment and may correspond to 800 kcal per treatment or 200 kcal/h.

In an alternative embodiment, shown in Figure 4, a plurality of nutritional bags 33', 33", 33''' are provided and each nutritional bag containing a component of the nutritional solution. A first bag 33' contains a solution of amino-acids, a second bag 33" contains a solution of glucose or dextrose, a third bag 33''' contains a solution of lipids. The three nutritional bags 33', 33", 33''' are each hanging on a respective weight scale 36', 36", 36''' and connected to a common nutritional line 34 provided with the infusion pump 35. In an alternative embodiment, not shown, each of the three nutritional bags 33', 33", 33''' may be coupled to a respective infusion pump.

According also to a method not falling under the claimed invention, the control unit 100 is programmed for receiving a patient prescription for the blood treatment, i.e. target values for the blood treatment and target values for the administration of the nutritional products to the patient. These target values may be entered manually, e.g. through the display touch screen 38 or a keyboard placed on the main body 37 of the apparatus 1. The prescription may alternatively be read from a patient card or from another kind of storage media device or received by the machine by means of a data transmission channel (wired or wireless).

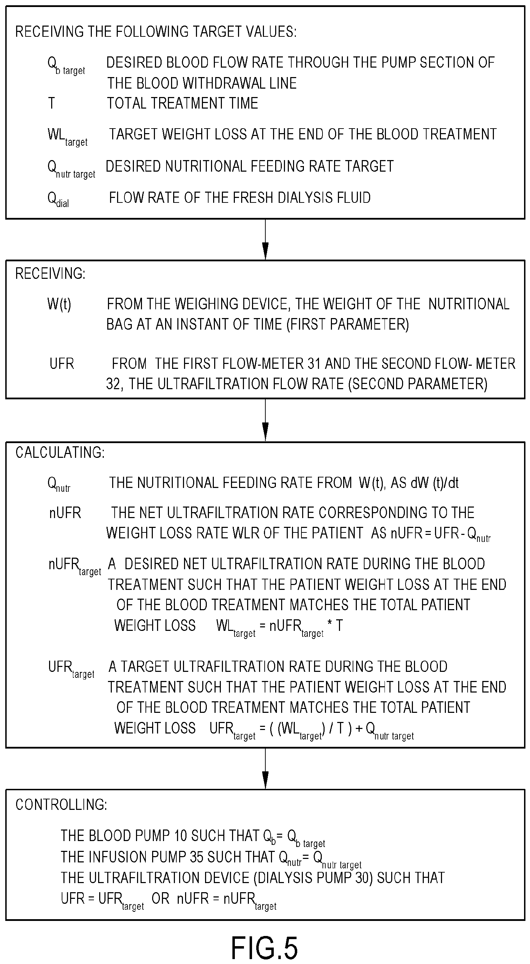

Example (Figure 5)

The control unit 100 is programmed for receiving as input the following target values:

| | |
|---|---|
| $Q_{b\ target}$ | Desired blood flow rate through the pump section of the blood withdrawal line |
| T | Total treatment time |
| $WL_{target}$ | Target weight loss at the end of the blood treatment |
| $Q_{nutr\ target}$ | Desired nutritional feeding rate target |
| $Q_{dial}$ | Flow rate of the fresh dialysis fluid |

The control unit 100 is programmed for receiving:

| | |
|---|---|
| W(t) | from the weighing device, the weight of the nutritional bag at an instant of time (first parameter) |
| UFR | from the first flow-meter 31 and the second flow-meter 32, the ultrafiltration flow rate (second parameter) |

The control unit 100 is programmed for calculating:

| | |
|---|---|
| $Q_{nutr}$ | the nutritional feeding rate from W(t), as $dW(t)/dt$ |
| nUFR | the net ultrafiltration rate corresponding to the weight loss rate WLR of the patient as $nUFR = UFR - Q_{nutr}$ |
| $nUFR_{target}$ | a desired net ultrafiltration rate during the blood treatment such that the patient weight loss at the end of the blood treatment matches the total patient weight loss |

$$WL_{target} = nUFR_{target} * T$$

$UFR_{target}$ a target ultrafiltration rate during the blood treatment such that the patient weight loss at the end of the blood treatment matches the total patient

$$weight\ loss\ WLR*T\ =\ nUFR*T\ =\ WL(T)\ =\ WL_{target}$$

$$WL_{target}\ =\ nUFR_{target}\ *\ T\ =\ UFR_{target}\ *\ T\ -\ Q_{nutr\ target}\ *\ T$$

$$UFR_{target}\ =\ ((WL_{target})/T)\ +\ Q_{nutr\ target}$$

The control unit 100 is programmed for controlling:

the blood pump 10 such that $Q_b = Q_{b\ target}$
the infusion pump 35 such that $Q_{nutr} = Q_{nutr\ target}$
the ultrafiltration device (dialysis pump 30) such that $UFR = UFR_{target}$ or $nUFR = nUFR_{target}$

In particular, the control unit 100 is programmed for controlling the ultrafiltration device (dialysis pump 30) such that the flow rate of the spent dialysis fluid $Q_{eff}$ is equal to $Q_{eff\ target}$ wherein

$$Q_{eff\ target}\ =\ UFR_{target}\ +\ Q_{dial}\ =\ ((WL_{target})/T)\ +\ Q_{nutr\ target}\ +\ Q_{dial}$$

Numerical example

| | |
|---|---|
| T | 4 hours |
| $WL_{target}$ | 3.5 kg = 3500 ml (blood density is very close to water density) |
| $Q_{nutr\ target}$ | 150 ml/hour = 2.5 ml/min |
| Qdial | 500 ml/min |

$$Q_{eff\ target}\ =\ ((WL_{target})/T)\ +\ Q_{nutr\ target}\ +\ Q_{dial}\ =\ 517\ ml/min$$

According to other embodiments, the target value entered directly into the control unit 100 may be, instead of the target weight loss rate $WLR_{target}$, the desired net ultrafiltration rate $nUFR_{target}$ (corresponding to the target weight loss rate $WLR_{target}$).

According to other embodiments, instead of the desired nutritional feeding rate target $Q_{nutr\ target}$, the control unit 100 is programmed for receiving as input a total amount of the nutritional solution $W_{nutr\ target}$ administered to the patient P at the end of the treatment time T. The control unit 100 is programmed for calculating the desired nutritional feeding rate target $Q_{nutr\ target}$ as $W_{nutr\ target}/T$ or to calculate a desired nutritional feeding rate target $Q_{nutr\ target}(t)$ which changes over time during treatment and such that the integration of $Q_{nutr\ target}(t)$ over time T is equal to $W_{nutr\ target}$. According to other embodiments, the control unit is programmed for integrating the ultrafiltration flow rate UFR (second parameter) received from the first flow-meter 31 and the second flow-meter 32, and by subtracting the weight W(t) of the nutritional bag (first parameter) from the integrated ultrafiltration rate UFR to calculate the patient weight loss (WL(t)) at time t. Then to compare the calculated patient weight loss (WL(t)) at time t with the target total weight loss at the end of the blood treatment $WL_{target}$ and to stop the treatment when the patient weight loss is equal to $WL_{target}$.

According to some embodiments, the nutritional feeding rate $Q_{nutr}$, the ultrafiltration flow rate UFR(t), the net ultrafiltration rate nUFR(t) and other related values are constant over time during blood treatment.

According to some embodiments, the feeding rate $Q_{nutr}$ of the nutritional solution, the ultrafiltration flow rate UFR(t), the net ultrafiltration rate nUFR(t) and other related values may be controlled to change over time during blood treatment. For instance, the net ultrafiltration rate nUFR(t) (or the weight loss rate WLR) may be greater at the start of the blood treatment, when the patient P may release more of liquid, than at the end. For instance, the nutritional feeding rate target $Q_{nutr}$ may be greater at the end of the blood treatment, where it is more likely that the nutritional solution is not eliminated through the blood treatment, than at the start.

The control unit 100 may be programmed for changing the feeding rate $Q_{nutr}$ of the nutritional solution by controlling the infusion pump 35 and/or for changing the ultrafiltration flow rate UFR(t) by controlling the dialysis pump 30.

The control unit 100 may also be programmed for storing data related to the nutritional solution administered during the extracorporeal blood treatment and to display on the display screen said data together with other values related to the blood treatment.

For instance, said data may comprise at least one of: the weight of the at least one nutritional bag, a feeding rate $Q_{nutr}$, an amount of nutritional solution administered at an instant of time, the total amount of the nutritional solution to be administered, a composition of the nutritional solution.

**Claims**

1. An apparatus for extracorporeal blood treatment for chronic therapy comprising:

   - a main body (37);
   - a filtration unit (2) having a primary chamber (3) and a secondary chamber (4) separated by a semi-permeable membrane (5);
   - a blood circuit coupled to the filtration unit (2) and comprising a blood withdrawal line (6) connected to an inlet (3a) of the primary chamber (3), a blood return line (7) connected to an outlet (3b) of the primary chamber (3), said blood withdrawal line (6) and blood return line (7) being configured for connection to a patient cardiovascular system;
   - a blood pump (10) configured to be coupled to a pump section of the blood circuit;
   - a dialysis circuit comprising a dialysis supply line (11), for a fresh dialysis fluid, connected to an inlet (4a) of the secondary chamber (4) and a dialysis effluent line (12), for a spent dialysis fluid, connected to an outlet (4b) of the secondary chamber (4);
   - a preparation device (13) for preparing the fresh dialysis fluid comprising a preparation line connected to a liquid source and at least one container of concentrate, wherein the container is located on a respective injection line predisposed to supply substances to the preparation line, wherein the preparation device (13) is connected to the dialysis supply line (11) and comprises a regulating device (14) for regulating the composition of the fresh dialysis fluid;
   - a nutritional solution comprising a mixture of protein, carbohydrate and fat;
   - at least one nutritional bag (33) containing the nutritional solution;
   - a nutritional line (34) having a first end in fluid communication with the nutritional bag (33) and a second end for infusing the nutritional solution into either the blood return line (7) or directly into the patient vascular system;
   - an infusion pump (35) coupled to the nutritional line (34) to deliver a feeding rate ($Q_{nutr}$) of the nutritional solution through the nutritional line (34), wherein the infusion pump is attached to the main body;
   - at least one sensing element (36) configured to provide a first signal related to an actual flow rate in the nutritional line;
   - an ultrafiltration device (30) configured to achieve a fluid removal from the patient through the semi-permeable membrane (5);
   - at least one sensor (31, 32) configured to provide a second signal related to an ultrafiltration rate (UFR), the at least one sensor (31, 32) comprises at least one flowmeter placed on at least one of the dialysis supply line (11) and the dialysis effluent line (12);
   - a control unit (100) connected at least to the ultrafiltration device (30) and to the infusion pump (35), to the sensing element (36)and to the at least one sensor (31, 32) and programmed for:

     ◦ receiving a patient prescription including at least one of:

       ▪ a total patient weight loss ($WL_{target}$) to be achieved at the end of the blood treatment and a total treatment time (T);
       ▪ a desired net ultrafiltration rate ($nUFR_{target}$);

     ◦ collecting from the sensing element (36) the first signal and determining a first parameter (W) related to the actual flow rate in the nutritional line (34), wherein the at least one sensing element (36) is a weighing device configured to provide a weight of the at least one nutritional bag (33) and wherein the control unit (100) is configured to receive a weight signal from the weighing device and to determine the first parameter (W) based on the weight variation over time of the nutritional bag, the first parameter being the actual flow rate in the nutritional line;
     ◦ collecting from the at least one sensor (31, 32) the second signal and determining a second parameter (FR) related to the ultrafiltration rate (UFR), wherein the second parameter (FR) is the ultrafiltration rate (UFR);
     ◦ controlling the infusion pump (35);
     ◦ controlling the ultrafiltration device (30) to achieve the patient prescription based on the first parameter (W) and on the second parameter (FR).

2. An apparatus for extracorporeal blood treatment for chronic therapy comprising:

- a main body (37);
- a filtration unit (2) having a primary chamber (3) and a secondary chamber (4) separated by a semi-permeable membrane (5) ;
- a blood circuit coupled to the filtration unit (2) and comprising a blood withdrawal line (6) connected to an inlet (3a) of the primary chamber (3), a blood return line (7) connected to an outlet (3b) of the primary chamber (3), said blood withdrawal line (6) and blood return line (7) being configured for connection to a patient cardiovascular system;
- a blood pump (10) configured to be coupled to a pump section of the blood circuit;
- a dialysis circuit comprising a dialysis supply line (11), for a fresh dialysis fluid, connected to an inlet (4a) of the secondary chamber (4) and a dialysis effluent line (12), for a spent dialysis fluid, connected to an outlet (4b) of the secondary chamber (4);
- a preparation device (13) for preparing the fresh dialysis fluid comprising a preparation line connected to a liquid source and at least one container of concentrate, wherein the container is located on a respective injection line predisposed to supply substances to the preparation line, wherein the preparation device (13) is connected to the dialysis supply line (11) and comprises a regulating device (14) for regulating the composition of the fresh dialysis fluid;
- balance chambers operatively coupled to the dialysis circuit to precisely balance fresh dialysis fluid prepared by the preparation device with spent dialysis fluid exiting the filtration unit;
- an ultrafiltration line and an ultrafiltration pump to remove spent dialysis fluid from upstream the balance chamber in the dialysis effluent line;
- a nutritional solution comprising a mixture of protein, carbohydrate and fat;
- at least one nutritional bag (33) containing the nutritional solution;
- a nutritional line (34) having a first end in fluid communication with the nutritional bag (33) and a second end for infusing the nutritional solution into either the blood return line (7) or directly into the patient vascular system;
- an infusion pump (35) coupled to the nutritional line (34) to deliver a feeding rate ($Q_{nutr}$) of the nutritional solution through the nutritional line (34), wherein the infusion pump is attached to the main body;
- at least one sensing element (36) configured to provide a first signal related to an actual flow rate in the nutritional line;
- an ultrafiltration device (30) configured to achieve a fluid removal from the patient through the semi-permeable membrane (5) ;
- at least one sensor (31, 32) sensing the amount of spent dialysis fluid removed by the ultrafiltration pump and configured to provide a second signal related to an ultrafiltration rate (UFR);
- a control unit (100) connected at least to the ultrafiltration device (30) and to the infusion pump (35), to the sensing element (36)and to the at least one sensor (31, 32) and programmed for:

  ◦ receiving a patient prescription including at least one of:

    ▪ a total patient weight loss ($WL_{target}$) to be achieved at the end of the blood treatment and a total treatment time (T);
    ▪ a desired net ultrafiltration rate ($nUFR_{target}$);

  ◦ collecting from the sensing element (36) the first signal and determining a first parameter (W) related to the actual flow rate in the nutritional line (34), wherein the at least one sensing element (36) is a weighing device configured to provide a weight of the at least one nutritional bag (33) and wherein the control unit (100) is configured to receive a weight signal from the weighing device and to determine the first parameter (W) based on the weight variation over time of the nutritional bag, the first parameter being the actual flow rate in the nutritional line;
  ◦ collecting from the at least one sensor (31, 32) the second signal and determining a second parameter (FR) related to the ultrafiltration rate (UFR);
  ◦ controlling the infusion pump (35);

controlling the ultrafiltration device (30) to achieve the patient prescription based on the first parameter (W) and on the second parameter (FR).

**3.** The apparatus of claim 1 or 2, wherein the control unit (12) is programmed for calculating and/or storing data related to the nutritional solution administered during the extracorporeal blood treatment; said data comprise at least one of: the weight of the at least one nutritional bag (33), the feeding rate ($Q_{nutr}$) of the nutritional solution through the nutritional line (34), an amount of nutritional solution administered at an instant of time, a total amount of the nutritional solution to be administered, a composition of the nutritional solution.

**4.** The apparatus of anyone of the preceding claims 1 to 3, wherein the feeding rate ($Q_{nutr}$) of the nutritional solution during blood treatment is between 50 ml/h and 500 ml/h .

**5.** The apparatus of anyone of the preceding claims 1 to 4, wherein the control unit (100) is programmed for changing the feeding rate ($Q_{nutr}$) of the nutritional solution.

**6.** The apparatus of anyone of the preceding claims 1 to 5, wherein the first parameter (W) is the weight (W(t)) of the nutritional bag (33) at an instant of time.

**7.** The apparatus of the preceding claim 1, wherein a net ultrafiltration rate (nUFR) is calculated by calculating a feeding rate ($Q_{nutr}$) of the nutritional solution from the weight (W(t)) of the nutritional bag (33) and by subtracting the feeding rate ($Q_{nutr}$) from the ultrafiltration rate (UFR); wherein the ultrafiltration device (30) is controlled so that the net ultrafiltration rate (nUFR) matches the desired net ultrafiltration rate ($nUFR_{target}$).

**8.** The apparatus of the preceding claim 1, wherein a patient weight loss (WL(t)) at an instant of time is calculated by integrating the ultrafiltration rate (UFR) at that instant of time and by subtracting the weight (W(t)) of the nutritional bag (33) at that instant of time from the integrated flow rate (UFR); wherein the ultrafiltration device (30) is controlled so that the patient weight loss (WL) at the end of the blood treatment matches the total patient weight loss ($WL_{target}$).

**9.** The apparatus of anyone of the preceding claims 1 to 8, wherein the nutritional line comprises a pump segment (39), a feeding tube segment (40) connected to an inlet of the pump segment (39), a delivery tube segment (41) connected to an outlet of the pump segment (39) and at least one rigid portion (44) comprising two pump connectors (44a) for receiving opposite ends of the pump segment (39), a delivery tube segment connector (44c) for receiving one end of the delivery tube segment (41) and a feeding tube segment connector (44b) for receiving one end of the feeding tube segment (40),
wherein the feeding tube segment and/or the delivery tube segment include/s a respective removable connector (42; 43), the removable connector (43) of the delivery tube segment (41) being connectable to a corresponding counter connector on the blood return line (7).

**10.** The apparatus of anyone of the preceding claim 3 or anyone of the preceding claim 4 to 9 when depending on claim 3, comprising a display screen (38) connected to the control unit (100), wherein the control unit (100) is programmed for displaying on said display screen (38) the data related to the nutritional solution.

**11.** The apparatus of anyone of the preceding claims, wherein the control unit (100) is contained in or supported by the main body (37); wherein the infusion pump (35) and the blood pump (10) are supported by the main body (37); wherein the blood circuit and the filtration unit (2) are disposable and are coupled in removable manner to the main body (37) and to the blood pump (10); wherein the nutritional line (34) is disposable and is coupled in removable manner to the main body (37) and to the infusion pump (35); wherein the dialysis circuit is not-disposable and is integrated in the main body (37).

**12.** The apparatus of the preceding claim 11, wherein the infusion pump (35) and the blood pump (10) are peristaltic pumps.

**13.** The apparatus of the preceding claim 11 or 12, wherein the ultrafiltration device (30) comprises at least one dialysis pump coupled to the dialysis supply line (11) and/or to the dialysis effluent line (12); wherein said at least one dialysis pump is mounted on the main body (37); said at least one dialysis pump (30) is a volumetric pump.

**Patentansprüche**

**1.** Vorrichtung zur extrakorporalen Blutbehandlung für chronische Therapie, umfassend:

- einen Hauptkörper (37);
- eine Filtrationseinheit (2) mit einer Primärkammer (3) und einer Sekundärkammer (4), die durch eine semipermeable Membran (5) getrennt sind;
- einen Blutkreislauf, der an die Filtrationseinheit (2) gekoppelt ist und eine Blutentnahmeleitung (6), die mit einem Einlass (3a) der Primärkammer (3) verbunden ist, und eine Blutrückführleitung (7) umfasst, die mit einem Auslass (3b) der Primärkammer (3) verbunden ist, wobei die Blutentnahmeleitung (6) und die Blutrückführleitung (7) zum Verbinden mit dem Herz-KreislaufSystem eines Patienten ausgestaltet sind;
- eine Blutpumpe (10), die ausgestaltet ist, um an einen Pumpenabschnitt des Blutkreislaufs gekoppelt zu werden;
- einen Dialysekreislauf, umfassend eine Dialysezuführleitung (11) für frisches Dialysefluid, die mit einem Einlass (4a) der Sekundärkammer (4) verbunden ist, und eine Dialyseabflussleitung (12) für verbrauchtes Dialysefluid, die mit einem Auslass (4b) der Sekundärkammer (4) verbunden ist;
- eine Vorbereitungsvorrichtung (13) zum Vorbereiten des frischen Dialysefluids, umfassend eine Vorbereitungsleitung, die mit einer Flüssigkeitsquelle verbunden ist, und mindestens einen Behälter für Konzentrat, wobei der Behälter sich an einer jeweiligen vorgeordneten Injektionsleitung befindet, um der Vorbereitungsleitung Substanzen zuzuführen, wobei die Vorbereitungsvorrichtung (13) mit der Dialysezuführleitung (11) verbunden ist und eine Regelungsvorrichtung (14) zum Regeln der Zusammensetzung des frischen Dialysefluids umfasst;
- eine Ernährungslösung, die eine Mischung aus Protein, Kohlenhydrat und Fett umfasst;
- mindestens einen Ernährungsbeutel (33), der die Ernährungslösung enthält;
- eine Ernährungsleitung (34) mit einem ersten Ende in Fluidkommunikation mit dem Ernährungsbeutel (33) und einem zweiten Ende zum Infundieren der Ernährungslösung in entweder die Blutrückführleitung (7) oder direkt in das Gefäßsystem des Patienten;
- eine Infusionspumpe (35), die an die Ernährungsleitung (34) gekoppelt ist, um eine Einspeiserate ($Q_{Nähr}$) der Ernährungslösung durch die Ernährungsleitung (34) abzugeben, wobei die Infusionspumpe an dem Hauptkörper befestigt ist;
- mindestens ein Abfühlelement (36), das ausgestaltet ist, um ein erstes Signal bereitzustellen, das mit einer tatsächlichen Flussrate in der Ernährungsleitung verknüpft ist;
- eine Ultrafiltrationsvorrichtung (30), die ausgestaltet ist, um eine Fluidentfernung aus dem Patienten durch die semipermeable Membran (5) hindurch zu erreichen;
- mindestens einen Sensor (31, 32), der ausgestaltet ist, um ein zweites Signal bereitzustellen, das mit einer Ultrafiltrationsrate (UFR) verknüpft ist, wobei der mindestens eine Sensor (31, 32) mindestens einen Flussmesser umfasst, der auf mindestens einer von der Dialysezuführleitung (11) und der Dialyseabflussleitung (12) platziert ist;
- eine Steuereinheit (100), die mindestens mit der Ultrafiltrationsvorrichtung (30) und der Infusionspumpe (35), mit dem Abfühlelement (36) und dem mindestens einen Sensor (31, 32) verbunden ist und programmiert ist zum:

  ◦ Empfangen einer Patientenverordnung, die mindestens eines der folgenden einschließt:

    ▪ einen Gesamtgewichtsverlust des Patienten ($WL_{Ziel}$), der am Ende der Blutbehandlung erreicht werden soll, und eine Gesamtbehandlungszeit (T);
    ▪ eine gewünschte Nettoultrafiltrationsrate ($nUFR_{Ziel}$);

  ◦ Erfassen des ersten Signals von dem Abfühlelement (36), und Bestimmen eines ersten Parameters (W), der mit der tatsächlichen Flussrate in der Ernährungsleitung (34) verknüpft ist, wobei das mindestens eine Abfühlelement (36) eine Wiegevorrichtung ist, die ausgestaltet ist, um ein Gewicht des mindestens einen Ernährungsbeutels (33) bereitzustellen, und wobei die Steuereinheit (100) ausgestaltet ist, um ein Gewichtssignal von der Wiegevorrichtung zu empfangen und den ersten Parameter (W) basierend auf der Gewichtsvariation des Ernährungsbeutels im Zeitverlauf zu bestimmen, wobei der erste Parameter die tatsächliche Flussrate in der Ernährungsleitung ist;
  ◦ Erfassen des zweiten Signals von dem mindestens einen Sensor (31, 32), und Bestimmen eines zweiten Parameters (FR), der mit der Ultrafiltrationsrate (UFR) verknüpft ist, wobei der zweite Parameter (FR) die Ultrafiltrationsrate (UFR) ist;
  ◦ Steuern der Infusionspumpe (35);
  ◦ Steuern der Ultrafiltrationsvorrichtung (30) zum Erreichen der Patientenverordnung basierend auf dem ersten Parameter (W) und dem zweiten Parameter (FR).

2. Vorrichtung zur extrakorporalen Blutbehandlung für chronische Therapie, umfassend:

- einen Hauptkörper (37);
- eine Filtrationseinheit (2) mit einer Primärkammer (3) und einer Sekundärkammer (4), die durch eine semipermeable Membran (5) getrennt sind;
- einen Blutkreislauf, der an die Filtrationseinheit (2) gekoppelt ist und eine Blutentnahmeleitung (6), die mit einem Einlass (3a) der Primärkammer (3) verbunden ist, und eine Blutrückführleitung (7) umfasst, die mit einem Auslass (3b) der Primärkammer (3) verbunden ist, wobei die Blutentnahmeleitung (6) und die Blutrückführleitung (7) zum Verbinden mit dem Herz-KreislaufSystem eines Patienten ausgestaltet sind;
- eine Blutpumpe (10), die ausgestaltet ist, um an einen Pumpenabschnitt des Blutkreislaufs gekoppelt zu werden;
- einen Dialysekreislauf, umfassend eine Dialysezuführleitung (11) für frisches Dialysefluid, die mit einem Einlass (4a) der Sekundärkammer (4) verbunden ist, und eine Dialyseabflussleitung (12) für verbrauchtes Dialysefluid, die mit einem Auslass (4b) der Sekundärkammer (4) verbunden ist;
- eine Vorbereitungsvorrichtung (13) zum Vorbereiten des frischen Dialysefluids, umfassend eine Vorbereitungsleitung, die mit einer Flüssigkeitsquelle verbunden ist, und mindestens einen Behälter für Konzentrat, wobei der Behälter sich an einer jeweiligen vorgeordneten Injektionsleitung befindet, um der Vorbereitungsleitung Substanzen zuzuführen, wobei die Vorbereitungsvorrichtung (13) mit der Dialysezuführleitung (11) verbunden ist und eine Regelungsvorrichtung (14) zum Regeln der Zusammensetzung des frischen Dialysefluids umfasst;
- Ausgleichen von Kammern, die operativ an den Dialysekreislauf gekoppelt sind, um Frischdialysefluid, das durch die Vorbereitungsvorrichtung vorbereitet wurde, genau mit verbrauchtem Dialysefluid, das die Filtrationseinheit verlässt, auszugleichen;
- eine Ultrafiltrationsleitung und eine Ultrafiltrationspumpe, um verbrauchtes Dialysefluid von stromaufwärts der Ausgleichkammer in der Dialyseabflussleitung zu entfernen;
- eine Ernährungslösung, die eine Mischung aus Protein, Kohlenhydrat und Fett umfasst;
- mindestens einen Ernährungsbeutel (33), der die Ernährungslösung enthält;
- eine Ernährungsleitung (34) mit einem ersten Ende in Fluidkommunikation mit dem Ernährungsbeutel (33) und einem zweiten Ende zum Infundieren der Ernährungslösung in entweder die Blutrückführleitung (7) oder direkt in das Gefäßsystem des Patienten;
- eine Infusionspumpe (35), die an die Ernährungsleitung (34) gekoppelt ist, um eine Einspeiserate ($Q_{Nähr}$) der Ernährungslösung durch die Ernährungsleitung (34) abzugeben, wobei die Infusionspumpe an dem Hauptkörper befestigt ist;
- mindestens ein Abfühlelement (36), das ausgestaltet ist, um ein erstes Signal bereitzustellen, das mit einer tatsächlichen Flussrate in der Ernährungsleitung verknüpft ist;
- eine Ultrafiltrationsvorrichtung (30), die ausgestaltet ist, um eine Fluidentfernung aus dem Patienten durch die semipermeable Membran (5) hindurch zu erreichen;
- mindestens einen Sensor (31, 32), der die Menge des verbrauchten Dialysefluids abfühlt, die durch die Ultrafiltrationspumpe entfernt wurde, und ausgestaltet ist, um ein zweites Signal bereitzustellen, das mit einer Ultrafiltrationsrate (UFR) verknüpft ist;
- eine Steuereinheit (100), die mindestens mit der Ultrafiltrationsvorrichtung (30) und der Infusionspumpe (35), mit dem Abfühlelement (36) und dem mindestens einen Sensor (31, 32) verbunden ist und programmiert ist zum:

   ◦ Empfangen einer Patientenverordnung, die mindestens eines der folgenden einschließt:

      ▪ einen Gesamtgewichtsverlust des Patienten ($WL_{Ziel}$), der am Ende der Blutbehandlung erreicht werden soll, und eine Gesamtbehandlungszeit (T);
      ▪ eine gewünschte Nettoultrafiltrationsrate ($nUFR_{Ziel}$);

   ◦ Erfassen des ersten Signals von dem Abfühlelement (36), und Bestimmen eines ersten Parameters (W), der mit der tatsächlichen Flussrate in der Ernährungsleitung (34) verknüpft ist, wobei das mindestens eine Abfühlelement (36) eine Wiegevorrichtung ist, die ausgestaltet ist, um ein Gewicht des mindestens einen Ernährungsbeutels (33) bereitzustellen, und wobei die Steuereinheit (100) ausgestaltet ist, um ein Gewichtssignal von der Wiegevorrichtung zu empfangen und den ersten Parameter (W) basierend auf der Gewichtsvariation des Ernährungsbeutels im Zeitverlauf zu bestimmen, wobei der erste Parameter die tatsächliche Flussrate in der Ernährungsleitung ist;
   ◦ Erfassen des zweiten Signals von dem mindestens einen Sensor (31, 32), und Bestimmen eines zweiten Parameters (FR), der mit der Ultrafiltrationsrate (UFR) verknüpft ist;
   ◦ Steuern der Infusionspumpe (35);
   ◦ Steuern der Ultrafiltrationsvorrichtung (30) zum Erreichen der Patientenverordnung basierend auf dem ersten Parameter (W) und dem zweiten Parameter (FR).

3. Vorrichtung nach Anspruch 1 oder 2, wobei die Steuereinheit (12) zum Berechnen und/oder Speichern von Daten programmiert ist, die mit der Ernährungslösung verknüpft sind, die während der extrakorporalen Blutbehandlung verabreicht wurde; wobei die Daten mindestens eines der folgenden umfassen: das Gewicht des mindestens einen Ernährungsbeutels (33), die Speiserate ($Q_{Nähr}$) der Ernährungslösung durch die Ernährungsleitung (34), eine Menge an Ernährungslösung, die in einem Zeitmoment verabreicht wird, eine Gesamtmenge der zu verabreichenden Ernährungslösung, eine Zusammensetzung der Ernährungslösung.

4. Vorrichtung nach einem der vorhergehenden Ansprüche 1 bis 3, wobei die Speiserate ($Q_{Nähr}$) der Ernährungslösung während der Blutbehandlung zwischen 50 ml/h und 500 ml/h liegt.

5. Vorrichtung nach einem der vorhergehenden Ansprüche 1 bis 4, wobei die Steuereinheit (100) programmiert ist, um die Speiserate ($Q_{Nähr}$) der Ernährungslösung zu verändern.

6. Vorrichtung nach einem der vorhergehenden Ansprüche 1 bis 5, wobei der erste Parameter (W) das Gewicht (W(t)) des Ernährungsbeutels (33) in einem Zeitmoment ist.

7. Vorrichtung nach dem vorhergehenden Anspruch 1, wobei eine Nettoultrafiltrationsrate (nUFR) berechnet wird, indem eine Speiserate ($Q_{Nähr}$) der Ernährungslösung aus dem Gewicht (W(t)) des Ernährungsbeutels (33) berechnet wird, und die Speiserate ($Q_{Nähr}$) von der Ultrafiltrationsrate (UFR) subtrahiert wird; wobei die Ultrafiltrationsvorrichtung (30) so gesteuert wird, dass die Nettoultrafiltrationsrate (nUFR) der gewünschten Nettoultrafiltrationsrate ($nUFR_{Ziel}$) entspricht.

8. Vorrichtung nach dem vorhergehenden Anspruch 1, wobei ein Patientengewichtsverlust (WL(t)) an einem Zeitmoment berechnet wird, indem die Ultrafiltrationsrate (UFR) an jenem Zeitmoment integriert wird und das Gewicht (W(t)) des Ernährungsbeutels (33) an jenem Zeitmoment von der integrierten Flussrate (UFR) subtrahiert wird; wobei die Ultrafiltrationsvorrichtung (30) so gesteuert wird, dass der Patientengewichtsverlust (WL) am Ende der Blutbehandlung dem Gesamtgewichtsverlust des Patienten ($WL_{Ziel}$) entspricht.

9. Vorrichtung nach einem der vorhergehenden Ansprüche 1 bis 8, wobei die Ernährungsleitung ein Pumpensegment (39), ein Speiseschlauchsegment (40), das mit einem Einlass des Pumpensegments (39) verbunden ist, ein Abgabeschlauchsegment (41), das mit einem Auslass des Pumpensegments (39) verbunden ist, und mindestens einen starren Abschnitt (44), der zwei Pumpenverbinder (44a) umfasst, um entgegengesetzte Enden des Pumpensegments (39) anzunehmen, einen Abgabeschlauchsegmentverbinder (44c) zum Annehmen eines Endes des Abgabeschlauchsegments (41) und einen Speiseschlauchsegmentverbinder (44b) zum Annehmen eines Endes des Speiseschlauchsegments (40) umfasst, wobei das Speiseschlauchsegment und/oder das Abgabeschlauchsegment einen jeweiligen entfernbaren Verbinder (42; 43) einschließt/einschließen, der entfernbare Verbinder (43) des Abgabeschlauchsegments (41) mit einem entsprechenden Gegenverbinder an der Blutrückführleitung (7) verbunden ist.

10. Vorrichtung nach einem der vorhergehenden Ansprüche 3 oder einem der vorhergehenden Ansprüche 4 bis 9 in Abhängigkeit von Anspruch 3, umfassend einen Anzeigebildschirm (38), der mit der Steuereinheit (100) verbunden ist, wobei die Steuereinheit (100) zum Anzeigen der Daten, die mit der Ernährungslösung verknüpft sind, auf dem Anzeigebildschirm (38) programmiert ist.

11. Vorrichtung nach einem der vorhergehenden Ansprüche, wobei die Steuereinheit (100) in dem Hauptkörper (37) enthalten ist oder von diesem getragen wird; wobei die Infusionspumpe (35) und die Blutpumpe (10) durch den Hauptkörper (37) getragen werden; wobei der Blutkreislauf und die Filtrationseinheit (2) zum einmaligen Gebrauch sind und in entfernbarer Weise an den Hauptkörper (37) und an die Blutpumpe (10) gekoppelt sind; wobei die Ernährungsleitung (34) zum einmaligen Gebrauch ist und in entfernbarer Weise an den Hauptkörper (37) und an die Infusionspumpe (35) gekoppelt ist; wobei der Dialysekreislauf nicht zum einmaligen Gebrauch ist und in den Hauptkörper (37) integriert ist.

12. Vorrichtung nach dem vorhergehenden Anspruch 11, wobei die Infusionspumpe (35) und die Blutpumpe (10) Peristaltikpumpen sind.

13. Vorrichtung nach dem vorhergehenden Anspruch 11 oder 12, wobei die Ultrafiltrationsvorrichtung (30) mindestens eine Dialysepumpe umfasst, die an die Dialysezuführleitung (11) und/oder an die Dialyseabflussleitung (12) gekoppelt ist; wobei die mindestens eine Dialysepumpe auf dem Hauptkörper (37) montiert ist; wobei die mindestens eine Dialysepumpe (30) eine volumetrische Pumpe ist.

## Revendications

1. Appareil de traitement sanguin extracorporel pour une thérapie chronique comprenant :

- un corps principal (37) ;
- une unité de filtration (2) ayant une chambre primaire (3) et une chambre secondaire (4) séparées par une membrane semi-perméable (5) ;
- un circuit sanguin couplé à l'unité de filtration (2) et comprenant une ligne de prélèvement de sang (6) reliée à une entrée (3a) de la chambre primaire (3), une ligne de retour de sang (7) reliée à une sortie (3b) de la chambre primaire (3), lesdites ligne de prélèvement de sang (6) et ligne de retour de sang (7) étant configurées pour être reliées au système cardiovasculaire d'un patient ;
- une pompe à sang (10) configurée pour être couplée à une section de pompe du circuit sanguin ;
- un circuit de dialyse comprenant une ligne d'alimentation de dialyse (11), pour un fluide de dialyse frais, reliée à une entrée (4a) de la chambre secondaire (4) et une ligne d'effluent de dialyse (12), pour un fluide de dialyse usé, reliée à une sortie (4b) de la chambre secondaire (4) ;
- un dispositif de préparation (13) pour préparer le fluide de dialyse frais comprenant une ligne de préparation reliée à une source de fluide et au moins un récipient de concentré, le récipient étant situé sur une ligne d'injection respective prédisposée pour fournir des substances à la ligne de préparation, le dispositif de préparation (13) étant relié à la ligne d'alimentation de dialyse (11) et comprenant un dispositif de régulation (14) pour réguler la composition du fluide de dialyse frais ;
- une solution nutritionnelle comprenant un mélange de protéines, de glucides et de lipides ;
- au moins une poche nutritionnelle (33) contenant la solution nutritionnelle ;
- une ligne nutritionnelle (34) ayant une première extrémité en communication fluidique avec la poche nutritionnelle (33) et une seconde extrémité pour perfuser la solution nutritionnelle soit dans la ligne de retour de sang (7) soit directement dans le système vasculaire du patient ;
- une pompe à perfusion (35) couplée à la ligne nutritionnelle (34) pour administrer un débit d'alimentation ($Q_{nutr}$) de la solution nutritionnelle à travers la ligne nutritionnelle (34), la pompe à perfusion étant fixée au corps principal ;
- au moins un élément de détection (36) configuré pour fournir un premier signal lié à un débit réel dans la ligne nutritionnelle ;
- un dispositif d'ultrafiltration (30) configuré pour réaliser une élimination de fluide du patient à travers la membrane semi-perméable (5) ;
- au moins un capteur (31, 32) configuré pour fournir un second signal lié à un débit d'ultrafiltration (UFR), l'au moins un capteur (31, 32) comprenant au moins un débitmètre placé sur au moins l'une de la ligne d'alimentation de dialyse (11) et de la ligne d'effluent de dialyse (12) ;
- une unité de commande (100) reliée au moins au dispositif d'ultrafiltration (30) et à la pompe à perfusion (35), à l'élément de détection (36) et à l'au moins un capteur (31, 32) et programmée pour :

  ◦ recevoir une prescription du patient comprenant au moins l'un des éléments suivants :

    une perte de poids totale du patient ($WL_{target}$) à atteindre à la fin du traitement sanguin et une durée totale de traitement (T) ;
    un débit d'ultrafiltration net souhaité ;

  ◦ collecter à partir de l'élément de détection (36) le premier signal et déterminer un premier paramètre (W) lié au débit réel dans la ligne nutritionnelle (34), l'au moins un élément de détection (36) étant un dispositif de pesée configuré pour fournir un poids de l'au moins une poche nutritionnelle (33) et l'unité de commande (100) étant configurée pour recevoir un signal de poids du dispositif de pesée et pour déterminer le premier paramètre (W) sur la base de la variation de poids dans le temps de la poche nutritionnelle, le premier paramètre étant le débit réel dans la ligne nutritionnelle ;
  ◦ collecter à partir de l'au moins un capteur (31, 32) le second signal et déterminer un second paramètre (FR) lié au débit d'ultrafiltration (UFR), le second paramètre (FR) étant le débit d'ultrafiltration (UFR) ;
  ◦ commander la pompe à perfusion (35) ;

◦ commander le dispositif d'ultrafiltration (30) pour réaliser la prescription du patient sur la base du premier paramètre (W) et du second paramètre (FR).

2. Appareil de traitement sanguin extracorporel pour une thérapie chronique comprenant :

- un corps principal (37) ;
- une unité de filtration (2) ayant une chambre primaire (3) et une chambre secondaire (4) séparées par une membrane semi-perméable (5) ;
- un circuit sanguin couplé à l'unité de filtration (2) et comprenant une ligne de prélèvement de sang (6) reliée à une entrée (3a) de la chambre primaire (3), une ligne de retour de sang (7) reliée à une sortie (3b) de la chambre primaire (3), lesdites ligne de prélèvement de sang (6) et ligne de retour de sang (7) étant configurées pour être reliées au système cardiovasculaire d'un patient ;
- une pompe à sang (10) configurée pour être couplée à une section de pompe du circuit sanguin ;
- un circuit de dialyse comprenant une ligne d'alimentation de dialyse (11), pour un fluide de dialyse frais, reliée à une entrée (4a) de la chambre secondaire (4) et une ligne d'effluent de dialyse (12), pour un fluide de dialyse usé, reliée à une sortie (4b) de la chambre secondaire (4) ;
- un dispositif de préparation (13) pour préparer le fluide de dialyse frais comprenant une ligne de préparation reliée à une source de fluide et au moins un récipient de concentré, le récipient étant situé sur une ligne d'injection respective prédisposée pour fournir des substances à la ligne de préparation, le dispositif de préparation (13) étant relié à la ligne d'alimentation de dialyse (11) et comprenant un dispositif de régulation (14) pour réguler la composition du fluide de dialyse frais ;
- des chambres d'équilibrage couplées de manière opérationnelle au circuit de dialyse pour équilibrer précisément le fluide de dialyse frais préparé par le dispositif de préparation avec le fluide de dialyse usé sortant de l'unité de filtration ;
- une ligne d'ultrafiltration et une pompe d'ultrafiltration pour éliminer le fluide de dialyse usé en amont de la chambre d'équilibrage dans la ligne d'effluent de dialyse ;
- une solution nutritionnelle comprenant un mélange de protéines, de glucides et de lipides ;
- au moins une poche nutritionnelle (33) contenant la solution nutritionnelle ;
- une ligne nutritionnelle (34) ayant une première extrémité en communication fluidique avec la poche nutritionnelle (33) et une seconde extrémité pour perfuser la solution nutritionnelle soit dans la ligne de retour de sang (7) soit directement dans le système vasculaire du patient ;
- une pompe à perfusion (35) couplée à la ligne nutritionnelle (34) pour administrer un débit d'alimentation ($Q_{nutr}$) de la solution nutritionnelle à travers la ligne nutritionnelle (34), la pompe à perfusion étant fixée au corps principal ;
- au moins un élément de détection (36) configuré pour fournir un premier signal lié à un débit réel dans la ligne nutritionnelle ;
- un dispositif d'ultrafiltration (30) configuré pour réaliser une élimination de fluide du patient à travers la membrane semi-perméable (5) ;
- au moins un capteur (31, 32) détectant la quantité de fluide de dialyse usé éliminé par la pompe d'ultrafiltration et configuré pour fournir un second signal lié à un débit d'ultrafiltration (UFR) ;
- une unité de commande (100) reliée au moins au dispositif d'ultrafiltration (30) et à la pompe à perfusion (35), à l'élément de détection (36) et à l'au moins un capteur (31, 32) et programmée pour :

◦ recevoir une prescription du patient comprenant au moins l'un des éléments suivants :

une perte de poids totale du patient ($WL_{target}$) à atteindre à la fin du traitement sanguin et une durée totale de traitement (T) ;
un débit d'ultrafiltration net souhaité ;

◦ collecter à partir de l'élément de détection (36) le premier signal et déterminer un premier paramètre (W) lié au débit réel dans la ligne nutritionnelle (34), l'au moins un élément de détection (36) étant un dispositif de pesée configuré pour fournir un poids de l'au moins une poche nutritionnelle (33) et l'unité de commande (100) étant configurée pour recevoir un signal de poids du dispositif de pesée et pour déterminer le premier paramètre (W) sur la base de la variation de poids dans le temps de la poche nutritionnelle, le premier paramètre étant le débit réel dans la ligne nutritionnelle ;
◦ collecter à partir de l'au moins un capteur (31, 32) le second signal et déterminer un second paramètre (FR) lié au débit d'ultrafiltration (UFR) ;
◦ commander la pompe à perfusion (35) ; commander le dispositif d'ultrafiltration (30) pour réaliser la prescription du patient sur la base du premier paramètre (W) et du second paramètre (FR).

3. Appareil selon la revendication 1 ou 2, l'unité de commande (12) étant programmée pour calculer et/ou stocker des données relatives à la solution nutritionnelle administrée pendant le traitement sanguin extracorporel ; lesdites données comprenant au moins l'un des éléments suivants : le poids de l'au moins une poche nutritionnelle (33), le débit d'alimentation ($Q_{nutr}$) de la solution nutritionnelle, à travers la ligne nutritionnelle (34), une quantité de solution nutritionnelle administrée à un instant temporel, une quantité totale de solution nutritionnelle à administrer, une composition de la solution nutritionnelle.

4. Appareil selon l'une quelconque des revendications précédentes 1 à 3, le débit d'alimentation ($Q_{nutr}$) de la solution nutritionnelle pendant le traitement du sang étant compris entre 50 ml/h et 500 ml/h.

5. Appareil selon l'une quelconque des revendications précédentes 1 à 4, l'unité de commande (100) étant programmée pour modifier le débit d'alimentation ($Q_{nutr}$) de la solution nutritionnelle.

6. Appareil selon l'une quelconque des revendications précédentes 1 à 5, le premier paramètre (W) étant le poids (W(t)) de la poche nutritionnelle (33) à un instant temporel.

7. Appareil selon la revendication précédente 1, un débit d'ultrafiltration net (nUFR) étant calculé en calculant un débit d'alimentation ($Q_{nutr}$) de la solution nutritionnelle à partir du poids (W(t)) de la poche nutritionnelle (33) et en soustrayant le débit d'alimentation ($Q_{nutr}$) du débit d'ultrafiltration (UFR) ; le dispositif d'ultrafiltration (30) étant commandé de sorte que le débit d'ultrafiltration net (nUFR) corresponde au débit d'ultrafiltration net souhaité ($nUFR_{target}$).

8. Appareil selon la revendication précédente 1, une perte de poids du patient (WL(t)) à un instant temporel étant calculée en intégrant le débit d'ultrafiltration (UFR) à cet instant temporel et en soustrayant le poids (W(t)) de la poche nutritionnelle (33) à cet instant temporel du débit intégré (UFR) ; le dispositif d'ultrafiltration (30) étant commandé de sorte que la perte de poids du patient (WL) à la fin du traitement sanguin corresponde à la perte de poids totale du patient ($WL_{target}$).

9. Appareil selon l'une quelconque des revendications précédentes 1 à 8, la ligne nutritionnelle comprenant un segment de pompe (39), un segment de tube d'alimentation (40) relié à une entrée du segment de pompe (39), un segment de tube d'administration (41) relié à une sortie du segment de pompe (39) et au moins une partie rigide (44) comprenant deux connecteurs de pompe (44a) pour recevoir des extrémités opposées du segment de pompe (39), un connecteur de segment de tube d'administration (44c) pour recevoir une extrémité du segment de tube d'administration (41) et un connecteur de segment de tube d'alimentation (44b) pour recevoir une extrémité du segment de tube d'alimentation (40), le segment de tube d'alimentation et/ou le segment de tube d'administration comprenant un connecteur amovible respectif (42 ; 43), le connecteur amovible (43) du segment de tube d'administration (41) pouvant être relié à un contre-connecteur correspondant sur la ligne de retour de sang (7).

10. Appareil selon l'une quelconque des revendications précédentes 3 ou selon l'une quelconque des revendications précédentes 4 à 9 lorsque dépendantes de la revendication 3, celui-ci comprenant un écran d'affichage (38) relié à l'unité de commande (100), l'unité de commande (100) étant programmée pour afficher sur ledit écran d'affichage (38) les données relatives à la solution nutritionnelle.

11. Appareil selon l'une quelconque des revendications précédentes, l'unité de commande (100) étant contenue dans ou supportée par le corps principal (37) ; la pompe à perfusion (35) et la pompe à sang (10) étant supportées par le corps principal (37) ; le circuit sanguin et l'unité de filtration (2) étant jetables et étant couplés de manière amovible au corps principal (37) et à la pompe à sang (10) ; la ligne nutritionnelle (34) étant jetable et étant couplée de manière amovible au corps principal (37) et à la pompe à perfusion (35) ; le circuit de dialyse n'étant pas jetable et étant intégré dans le corps principal (37).

12. Appareil selon la revendication précédente, 11, la pompe à perfusion (35) et la pompe à sang (10) étant des pompes péristaltiques.

13. Appareil selon la revendication précédente 11 ou 12, le dispositif d'ultrafiltration (30) comprenant au moins une pompe de dialyse couplée à la ligne d'alimentation de dialyse (11) et/ou à la ligne d'effluent de dialyse (12) ; ladite au moins une pompe de dialyse étant montée sur le corps principal (37) ; ladite au moins une pompe de dialyse (30) étant une pompe volumétrique.

FIG.1

FIG.1A

FIG.2

FIG.3

FIG.4

RECEIVING THE FOLLOWING TARGET VALUES:

$Q_{b\ target}$      DESIRED BLOOD FLOW RATE THROUGH THE PUMP SECTION OF THE BLOOD WITHDRAWAL LINE

T      TOTAL TREATMENT TIME

$WL_{target}$      TARGET WEIGHT LOSS AT THE END OF THE BLOOD TREATMENT

$Q_{nutr\ target}$      DESIRED NUTRITIONAL FEEDING RATE TARGET

$Q_{dial}$      FLOW RATE OF THE FRESH DIALYSIS FLUID

↓

RECEIVING:

W(t)      FROM THE WEIGHING DEVICE, THE WEIGHT OF THE NUTRITIONAL BAG AT AN INSTANT OF TIME (FIRST PARAMETER)

UFR      FROM THE FIRST FLOW-METER 31 AND THE SECOND FLOW- METER 32, THE ULTRAFILTRATION FLOW RATE (SECOND PARAMETER)

↓

CALCULATING:

$Q_{nutr}$      THE NUTRITIONAL FEEDING RATE FROM W(t), AS dW (t)/dt

nUFR      THE NET ULTRAFILTRATION RATE CORRESPONDING TO THE WEIGHT LOSS RATE WLR OF THE PATIENT AS $nUFR = UFR - Q_{nutr}$

$nUFR_{target}$      A DESIRED NET ULTRAFILTRATION RATE DURING THE BLOOD TREATMENT SUCH THAT THE PATIENT WEIGHT LOSS AT THE END OF THE BLOOD TREATMENT MATCHES THE TOTAL PATIENT WEIGHT LOSS $WL_{target} = nUFR_{target} * T$

$UFR_{target}$      A TARGET ULTRAFILTRATION RATE DURING THE BLOOD TREATMENT SUCH THAT THE PATIENT WEIGHT LOSS AT THE END OF THE BLOOD TREATMENT MATCHES THE TOTAL PATIENT WEIGHT LOSS $UFR_{target} = ( (WL_{target}) / T ) + Q_{nutr\ target}$

↓

CONTROLLING:

THE BLOOD PUMP 10 SUCH THAT $Q_b = Q_{b\ target}$
THE INFUSION PUMP 35 SUCH THAT $Q_{nutr} = Q_{nutr\ target}$
THE ULTRAFILTRATION DEVICE (DIALYSIS PUMP 30) SUCH THAT
$UFR = UFR_{target}$ OR $nUFR = nUFR_{target}$

## FIG.5

**EP 3 892 313 B1**

## REFERENCES CITED IN THE DESCRIPTION

**Patent documents cited in the description**

- US 5776345 A
- US 2012143116 A
- EP 2644215 A